# FASCICULE DE BREVET EUROPEEN

(11) **EP 3 014 159 B1**
(45) Date de publication et mention de la délivrance du brevet: **26.07.2017**
(21) Numéro de dépôt: 14750539.0
(22) Date de dépôt: 24.06.2014
(51) Int. Cl.: F16L 33/025, F16L 33/22, F16L 37/098, A61J 1/14, A61M 39/10, A61M 39/12

(54) **CONNECTEUR FLUIDIQUE AVEC COLLIER ET PROTECTION**
FLUIDSTECKER MIT KLEMME UND SCHUTZ
FLUID CONNECTOR WITH CLAMP AND PROTECTION

(30) Priorité: 28.06.2013 FR 1356351
(43) Date de publication de la demande: 04.05.2016
(73) Titulaire: SARTORIUS STEDIM FMT SAS, 13400 Aubagne (FR)
(72) Inventeur: BLAKE, Florian, F-83400 Hyeres (FR); GIBELIN, Jérémy, F-83330 Le Beausset (FR); GAY, Isabelle, F-13124 Peypin (FR)
(74) Mandataire: Derambure Conseil
(86) Numéro de dépôt international: PCT/FR2014/051579
(87) Numéro de publication internationale: WO 2014/207370

(56) Documents cités:
- EP-A1- 2 143 986
- EP-A1- 2 341 273
- EP-A1- 2 497 989
- WO-A1-03/074918
- DE-U1-202009 000 508
- FR-A1- 2 385 971
- US-A- 4 049 034

## Description

L'invention concerne les connecteurs fluidiques, en particulier pour les connexions ou raccordements fluidiques permettant de raccorder une conduite fluide à une autre conduite ou à un récipient, dans le domaine des applications biopharmaceutiques.

Plus précisément, les conduites ou tubes utilisés dans le domaine biopharmaceutique sont des tubes souples, voire très souples, qui servent à transporter des substances biopharmaceutiques diverses, avec le plus souvent des précautions d'asepsie nécessaires.

Dans les applications biopharmaceutiques, ce type de tube souple permet la circulation, le passage, la communication d'un fluide, tel qu'un fluide biopharmaceutique et peut être raccordé soit à un tube souple similaire soit à un récipient ou un contenant, qui peut être rigide ou souple lui aussi.

Le récipient ou contenant en question peut être en l'espèce un récipient de stockage et/ou de traitement d'un contenu tel qu'un produit biopharmaceutique. Un tel récipient s'entend en l'espèce d'un récipient rigide ou semi rigide réutilisable ou d'un récipient souple à usage unique tel qu'une poche, ou même une cartouche filtrante.
Cette poche peut être une poche dite 2D, substantiellement peu épaisse, telle que celle commercialisée par Sartorius Stedim Biotech sous la marque Flexboy®, dont le volume, typiquement, peut être compris entre 50 millilitres et 50 litres. Cette poche souple peut aussi être une poche dite 3D, telle que celle commercialisée par Sartorius Stedim Biotech sous la marque Flexel®, avec un plus grand volume et une taille substantielle dans les trois dimensions. Il faut noter qu'un tube tel que celui auquel s'applique l'invention peut être interposé entre deux poches ou un plus grand nombre de poches.

Un tube tel que celui auquel s'applique l'invention, habituellement de section circulaire, est réalisé typiquement en matière plastique telle que le silicone, les élastomères thermoplastiques (TPE), mais également le PVC, la liste n'étant pas limitative. Il présente une certaine tenue d'ensemble et, simultanément, à la fois une certaine flexibilité d'ensemble et une certaine flexibilité locale, ce qui permet, moyennant l'exercice d'une force suffisante, de pincer le tube ou de le déformer substantiellement radialement.

Dans une réalisation typique, par exemple, le tube a un diamètre extérieur compris entre par exemple 8 millimètres et 30 millimètres, l'épaisseur dépendant du matériau, du diamètre et des applications.

Dans l'art connu, pour accoupler un tel tube souple, on enfile celui-ci sur un embout tubulaire, après quoi on place un collier de serrage autour du tube, puis on procède au serrage du collier. Ainsi, le collier serré exerce une pression radiale vers l'intérieur pour maintenir le tube souple sur l'embout, d'une part pour assurer une bonne étanchéité du tube vis-à-vis de l'embout et d'autre part pour éviter qu'une traction sur le tube ne conduise à un désengagement du tube de l'embout.

S'agissant de tels colliers de serrage, on peut utiliser par exemple un collier en matière plastique, de type polyamide par exemple en Rilsan®. Ce type de collier plastique, aussi parfois appelé Serflex®, comprend un système de crans sur une bande qui coopèrent avec un crochet à verrouillage agencé dans la tête, de manière à ce que le serrage ne soit pas réversible. En d'autres termes, une fois que l'on a engagé la bande dans la tête pour former une boucle, on tire sur la bande pour réduire le diamètre de la boucle et serrer le collier, le retour en arrière étant empêché par le crochet à verrouillage en prise dans un des crans de la bande. Après serrage, pour éviter que la bande ne dépasse trop du diamètre de la boucle du collier, on sectionne la portion libre de bande à proximité de la tête du collier. La partie restante non détachée de la bande présente souvent une arête vive qui peut s'avérer tranchante.

En alternative au collier plastique, on peut aussi utiliser un collier métallique qui se présente sous la forme d'un anneau préformé muni d'une ou deux oreilles faisant saillie vers l'extérieur relativement à la forme générale de l'anneau du collier, ce type de collier est parfois appelé collier Oetiker®. Après insertion du collier sur le tube à maintenir, on procède au moyen d'un outil au pincement de l'oreille (ou des oreilles) du collier ce qui provoque une déformation rémanente et ainsi un rétrécissement du diamètre principal de l'anneau et par conséquent serrage du collier sur le tube. Ce type de serrage par anneau métallique est particulièrement robuste et fiable. Toutefois, à l'endroit de l'oreille pincée par l'outil, il peut se présenter une aspérité ou une bavure qui forme une arête vive qui peut s'avérer agressive.
Le collier métallique pourrait être aussi un collier de type à rétreint serti, sans oreille. Lorsque de tels colliers, qu'ils soient plastiques ou métalliques, sont installés dans des ensembles biopharmaceutiques, ces derniers peuvent être amenés à être transportés ou déplacés, et par conséquent il existe un risque d'agression par des parties agressives de ces colliers vis-à-vis d'autres éléments de l'ensemble biopharmaceutique, en particulier des poches souples ou des tubes souples, ce qui peut entraîner une fuite ou une perte de stérilisation préjudiciable à l'application biopharmaceutique.
De plus, ces colliers sont faciles d'accès (et donc peuvent être démontés) et ne permettent pas de garantir une image et une esthétique satisfaisantes. Le document EP 2 341 273 A1 divulgue un dispositif de connexion selon le préambule de la revendication 1.

Il existe par conséquent le besoin de proposer une amélioration destinée à pallier, au moins en partie, l'inconvénient précité de l'art antérieur connu.
Ci-après, un exposé de l'invention telle que caractérisée dans les revendications. Selon un premier aspect, l'invention a pour objet un dispositif de connexion fluidique apte et destiné à raccorder une première paroi délimitant un premier espace fluidique, en forme de tube souple, à une seconde paroi délimitant un second espace fluidique, en forme de tube ou d'enceinte souple ou rigide à usage unique, de sorte à être apte et destiné à assurer une communication fluidique entre le premier espace fluidique et le second espace fluidique, comprenant :
- un premier connecteur délimitant un premier passage creux, apte et destiné à être raccordé à la première paroi et en communication fluidique avec le premier espace, le premier connecteur comprenant un embout tubulaire apte et destiné à recevoir le tube par insertion selon l'axe A,
- un collier de serrage étant apte et destiné à être disposé autour de l'extrémité du tube pour serrer ledit tube sur l'embout tubulaire du premier connecteur,
- un capot de protection formant une pièce distincte, apte à être clipsé dans une position active où ledit capot se trouve au moins partiellement autour du premier connecteur et de son collier de serrage, le capot de protection étant clipsé après serrage du collier, selon une orientation quelconque autour de l'axe A,
moyennant quoi le collier de serrage ne peut pas entrer en contact directement avec des éléments externes.

Moyennant quoi on évite ainsi un endommagement possible des poches souples ou des tubes disposés au voisinage immédiat par une partie agressive du collier de serrage. De plus, il est possible d'utiliser un capot de protection standard pour une variété de différents types de premier connecteur, que ce premier connecteur soit prévu pour être soudé à une poche ou prévu pour s'interfacer avec un second connecteur.
On remarque en outre que l'insertion du collier ne nécessite pas une orientation particulière autour d'un axe, ce qui rend cette opération relativement aisée à effectuer par un opérateur, même si celui-ci porte des équipements de protection aseptique comme des gants, une charlotte, des lunettes etc..

Selon une réalisation, le capot de protection est formé par deux demi-portions configurées pour se refermer l'une sur l'autre dans la direction radiale autour du collier de serrage. Moyennant quoi on peut avantageusement approvisionner et installer le capot de protection après l'insertion du tube sur l'embout.

Selon une réalisation, le capot de protection comprend deux portions hémicylindriques complémentaires reliées par une portion souple de charnière, et les deux portions étant aptes à être clipsées l'une à l'autre dans une zone diamétralement opposée à la zone de charnière. Ainsi, le capot de protection est avantageusement obtenu intégralement par une seule opération de moulage, et de plus son installation s'avère particulièrement aisée.

Selon une réalisation, le capot de protection comprend des pattes radiales flexibles destinées à venir s'appuyer sur le tube souple, dans une zone entre la position du collier et l'extrémité avant du tube. De sorte qu'une pression radiale peut être exercée par les pattes radiales flexibles en vue de maintenir le capot de protection dans sa position radialement et d'une certaine façon par frottement le long de l'axe.

Selon une réalisation, le capot de protection comprend des cloisons radiales pour encadrer le collier de serrage et ainsi immobiliser ou limiter le mouvement du capot le long de la direction axiale. Moyennant quoi on utilise le collier comme élément de positionnement du capot de protection sans faire appel à une autre forme du premier connecteur.

Selon une réalisation, le capot de protection est monobloc de révolution, le capot de protection étant disposé en attente en arrière sur le tube avant l'insertion du tube sur l'embout tubulaire, puis déplacé axialement vers l'avant pour venir dans une position active où il recouvre le collier de serrage. De sorte qu'il suffit de vérifier la position axiale du capot pour s'assurer de la protection vis-à-vis du collier. Le capot étant placé en attente sur le tube, cela permet de rationaliser le processus d'assemblage ; on peut aussi envisager une automatisation de la pose du capot de protection.

Selon une réalisation, le capot de protection comporte une portion avant apte à être clipsée sur une collerette du premier connecteur dans la position active du capot de protection du collier. On peut ainsi sécuriser la position du capot de protection dans sa position active sur la collerette entre un épaulement et un clip du capot.

Selon une réalisation, le capot de protection comporte une portion arrière apte à venir en appui sur le tube au voisinage de l'extrémité de l'embout du connecteur dans la position active du capot. Moyennant quoi on évite que des corps étrangers s'introduisent entre le tube et le capot de protection, et ceci permet de procurer un maintien complémentaire du capot de protection dans sa position active.

Selon une réalisation, le dispositif comprend en outre un second connecteur délimitant un second passage creux, apte et destiné à être raccordé à la seconde paroi et en communication fluidique avec le second espace,
le premier connecteur et le second connecteur étant aptes et destinés à être couplés mutuellement dans une position de couplage relative selon un axe A, et le capot de protection est apte à être clipsé au moins partiellement autour du premier connecteur et autour du deuxième connecteur, pour former témoin de bon couplage. De sorte que la bonne installation capot de protection reflète le bon couplage des premiers et seconds connecteurs.

Selon une réalisation, le capot comprend une rainure intérieure annulaire qui forme en position de couplage un moyen de verrouillage immobilisant des collerettes adjacentes appartenant respectivement aux premier et deuxième connecteurs.

Moyennant quoi on bénéficie d'un effet de verrouillage des premier et second connecteurs dans leur position de couplage au moyen de l'installation du capot de protection. Et on peut ainsi améliorer la fonction de témoin de bon montage.

Selon une réalisation, la rainure interne annulaire forme au moins une portion tronconique (de section de forme trapézoïdale) de manière à exercer une pression axiale sur les collerettes, ce qui tend à rapprocher les deux connecteurs. De sorte que l'on bénéficie du mouvement de clipsage du capot de protection pour terminer le cas échéant la course d'accouplement des premier et second connecteurs.

Selon une réalisation, le second connecteur comprend au moins une languette flexible adaptée pour venir se clipser sur une seconde collerette du premier connecteur de manière à former ainsi un premier verrouillage de la position de couplage préalable à l'installation du capot de protection. Moyennant quoi la position de couplage des deux connecteurs est conservée avant l'installation effective du capot de protection, nonobstant la réaction de joint.

Selon une réalisation, le dispositif peut comprendre en outre un identifiant de type code barre ou étiquette RFID ou code couleur disposé sur le capot de protection ou sur le premier connecteur. Moyennant quoi l'accès à des informations relatives à la poche souple et/ou au produit biopharmaceutique y contenu est aisé et facilite le processus de traçabilité.

Selon une réalisation, le capot comprend une lumière permettant de vérifier la présence du collier une fois le capot installé ; de sorte que l'on peut vérifier la position correcte du collier, y compris après l'installation du capot de protection.

Selon un deuxième aspect, l'invention a pour objet un ensemble biopharmaceutique comprenant un dispositif de connexion fluidique tel que décrit ci-dessus.

Selon un troisième aspect, l'invention vise aussi un kit de pièces comprenant un premier connecteur tel que décrit ci-dessus, un collier de serrage et un capot de protection tels que décrit ci-dessus pour fixer un tube souple sur le premier connecteur.

En outre l'invention vise un assemblage des pièces ci-dessus dans un état assemblé avec le collier de serrage protégé par le capot de protection.

On décrit maintenant brièvement les figures des dessins.
La figure 1 est une vue éclatée d'un dispositif de connexion conforme à la présente invention, avec une alternative concernant le premier connecteur qui peut être soit (A1) une interface directe fixée sur une poche ou bien (A2) destinée à s'interfacer avec un second connecteur.
La figure 2 est une vue en coupe axiale partielle du dispositif de connexion de la figure 1, avec le capot de protection en position active.
La figure 3 est une vue en perspective du capot de protection radiale en position ouverte, vue depuis une direction opposée par rapport à la figure 1.
La figure 4A est une vue en coupe axiale d'un dispositif de connexion selon un second mode de réalisation, avec un capot de protection à translation axiale.
La figure 4B montre une vue en perspective du capot de protection de la figure 4A.
La figure 5 est une vue en coupe axiale d'un dispositif de connexion selon un troisième mode de réalisation, dans lequel le premier connecteur s'interface avec le second connecteur, avec un autre mode de réalisation pour le capot de protection.

Ci-après un exposé détaillé de plusieurs modes de réalisation de l'invention assorti d'exemples et de référence aux dessins.

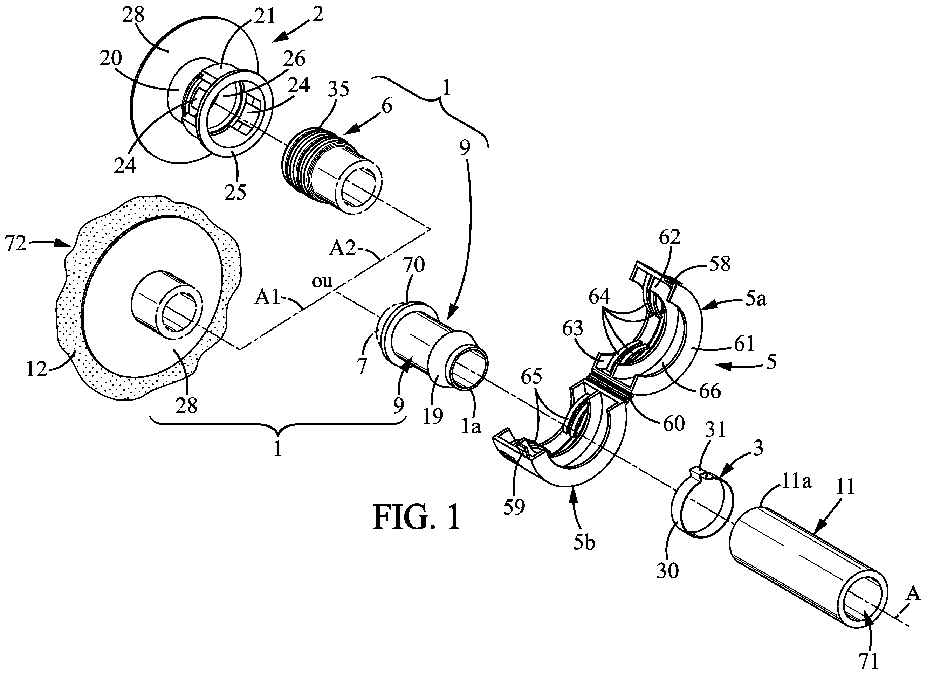

Dans l'exemple illustré aux figures 1-3, il s'agit de raccorder un tube souple **11** à un premier connecteur **1**, de façon étanche, au moyen d'un collier de serrage 3 sur un embout 9 du tube.
Le premier connecteur est destiné à être accouplé soit d'une part (Fig.1, alternative A1) directement à une poche au moyen d'un disque annulaire **28** soit d'autre part (Fig. 1, alternative A2) à un second connecteur **2** ceci afin de former une connexion fluide entre deux espaces fluidiques **71,72**.
Le tube souple 11 peut être défini généralement comme une première paroi 11 délimitant un premier espace fluidique **71**.
Le diamètre extérieur du tube souple 11 au repos est référencé **D2**, et on peut remarquer que le diamètre intérieur **D1** de l'embout tubulaire **9** est sensiblement voisin du diamètre intérieur du tube souple 11 au repos. Toutefois, plusieurs types de tube de diamètre différent peuvent être utilisés pour être insérés sur l'embout 9.

Le **premier connecteur 1** est réalisé en matière synthétique, plus précisément il peut être obtenu par moulage d'une matière plastique, par exemple polypropylène, du polyéthylène, du polycarbonate, du polysulfone, du polyamide, du PBT ou tout autre matériau plastique adapté.

Selon l'alternative **A2**, le premier connecteur **1** comprend un embout tubulaire **9** du côté de l'une de ses extrémités **1a**, et une interface de couplage **6** avec un second connecteur **2** à l'autre de ses extrémités. Selon l'alternative **A1**, le premier connecteur **1** comprend un disque annulaire **28** de raccordement direct à une poche.

L'embout tubulaire **9** est de révolution autour de l'axe **A**, et il en est de même de l'interface de couplage **6**. L'interface de couplage **6** est une interface de type mâle qui est prévue pour être insérée dans une interface de type femelle 2 décrite plus loin. Mais bien sûr, le contraire serait possible, c'est-à-dire une interface mâle dans le second connecteur et une interface femelle dans le premier connecteur. On pourrait aussi utiliser des interfaces asexuées.

Dans l'exemple présenté, l'interface mâle **6** dans le premier connecteur est généralement cylindrique de révolution autour de A, avec une ou deux rainures extérieures destinées à recevoir un ou deux joints toriques **35**.

Le corps **7** du connecteur comprend en partant de l'extrémité de l'embout 1a, une première collerette **70** en forme d'anneau cette première collerette sert de butée d'arrêt pour l'insertion du tube. Si le premier connecteur comporte une interface de raccordement avec le second connecteur alors une seconde collerette **14** peut être présente et son utilité sera vue plus loin.

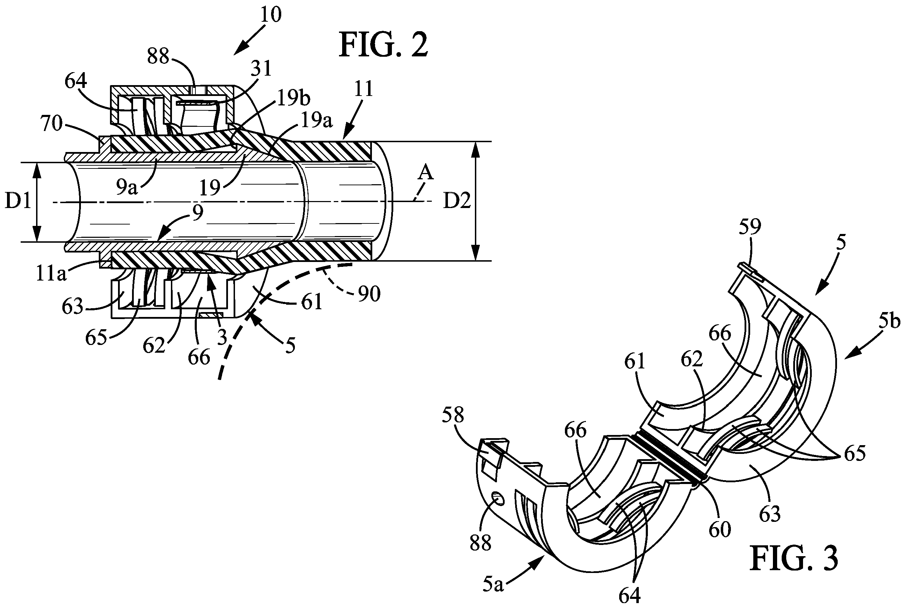

L'embout tubulaire **9** comprend un bourrelet annulaire **19**, qui dans l'exemple illustré présente une rampe douce **19a** du côté du tube souple à insérer et un épaulement **19b** du côté opposé. L'embout tubulaire pourrait comprendre un nombre plus grand de bourrelets comme par exemple des crans successifs comme connu en soi. Lorsque l'on enfile le tube souple **11** sur l'embout tubulaire **9**, le tube se déforme radialement vers l'extérieur grâce à la forme de rampe **19a**, puis au fur et à mesure de l'insertion, il retrouve un diamètre plus étroit au niveau de la surface de portée 9a cylindrique.
L'insertion peut se poursuivre jusqu'à ce que l'extrémité avant du tube **11a** vienne porter contre la première collerette **70** susmentionnée (voir figure 2).

Une fois que le tube souple est inséré sur l'embout tubulaire 9, on vient placer un **collier de serrage 3** autour du tube au niveau de la surface de portée 9a susmentionnée. Il faut noter ici que le collier de serrage **3** peut être disposé préalablement en attente autour d'une partie arrière du tube avant l'opération d'insertion.
Une fois que le collier est dans la position adéquate en vis-à-vis de la collerette 70 de l'embout tubulaire, on procède au serrage du collier.
Le collier de serrage représenté aux figures est un collier de type métallique avec une seule oreille **31** prévue pour le serrage. Toutefois il pourrait y avoir plus d'une oreille.

On utilise par exemple une pince pour venir écraser la forme en oreille **31** de manière à diminuer le diamètre de l'anneau **30** formé par le collier de serrage 3. De ce fait, le collier de serrage a alors un diamètre plus petit que celui de la surface extérieure du tube souple au repos, il exerce donc une pression radiale dirigée vers l'intérieur. Cette pression radiale a deux objectifs : le premier est d'assurer étanchéité suffisamment performante entre le tube **11** et l'embout tubulaire **9**, et le deuxième consiste à maintenir mécaniquement le tube autour de l'embout pour éviter qu'une traction exercée sur le tube ne conduise à désengager le tube de l'embout tubulaire, grâce à l'épaulement 19b susvisé.
Bien qu'on ait représenté un collier métallique à oreille, le collier pourrait être un collier de type plastique ou un collier métallique sans oreille de type à rétreint serti. En outre, il pourrait y avoir deux colliers côte à côte.

Selon l'alternative **A1**, le premier connecteur **1** comprend un disque **28** de fixation à la poche souple **12**, une telle fixation étant habituellement obtenue par une ou plusieurs soudures **29** ou bien par collage. Le disque **28** est plan et vient se plaquer sur une surface importante sur la poche **12**. Le centre du disque **28** est évidé pour établir une communication fluide entre l'espace cylindrique interne **82** du second connecteur et l'espace interne **72** de la poche.

Selon l'alternative **A2**, le premier connecteur **1** comprend une zone tubulaire **20** formant la partie femelle d'accouplement et recevant ainsi l'interface mâle **6** susmentionnée ; en position accouplée, les joints toriques **35** susmentionnés sont pressés vers l'intérieur par la surface cylindrique intérieure 26 de la zone tubulaire 20 formant l'interface femelle. On obtient ainsi un raccordement fluide étanche entre le premier connecteur 1 et le second connecteur 2.
De plus, le second connecteur 2 comprend un **corps 21** qui s'étend depuis la zone tubulaire 20 dans une direction opposée à la collerette 28, avec optionnellement des languettes de clipsage qui seront détaillées plus loin.

Après l'installation du collier, on procède à l'installation d'un **capot de protection 5**, dont la définition convient pour les alternatives A1 et A2 c'est-à-dire quel que soit la configuration du connecteur du côté opposé à l'embout.
Ce capot de protection **5** se présente sous la forme d'une pièce distincte du premier connecteur. Dans le premier mode de réalisation, il s'agit d'un capot à insertion radiale que l'on peut amener et installer après que le tube ait été inséré sur l'embout. Plus précisément, le capot de protection est formé par **deux demi-portions 5a,5b** configurées pour se refermer l'une sur l'autre dans la direction radiale autour du collier de serrage. Avantageusement, on peut prévoir de former les deux demi-portions intégralement par moulage d'une seule pièce, les deux demi-portions étant alors reliées par une zone de charnière **60**.
Il n'est toutefois pas exclu d'avoir deux demi-portions fournies séparément.
On remarque de plus que le capot de protection 5 peut être amené radialement depuis n'importe quelle direction, il n'y a pas d'orientation particulière à respecter autour de l'axe A.

Le capot de protection 5 est formé en matière plastique, par exemple du polypropylène, du polyéthylène, du polycarbonate, du polysulfone du polyamide, du PBT, de l'ABS.
Dans le capot de protection, il est prévu une gorge **66** intérieure assez large pour recevoir le collier de serrage et en particulier son oreille 31 en projection radiale. Cette gorge est encadrée par deux cloisons radiales **61,62**, qui permettent avantageusement de limiter le déplacement axial du capot après que celui-ci ait été installé en position active sur le collier de serrage. En effet, la partie centrale des cloisons radiales est en quasiment au contact du tube, elles encadrent donc le corps du collier et aussi l'oreille, et butent dans ledit collier en cas de mouvement axial du capot de protection.
En outre, le capot de protection 5 comporte des pattes radiales flexibles **64,65** prévues pour exercer une pression radiale vers l'intérieur sur le tube souple. Les pattes radiales flexibles sont positionnées en avant de la gorge **66** de manière à ce qu'elle vienne porter sur le tube dans une zone entre la position du collier et l'extrémité avant du tube **11a** (la première collerette **70**).

De plus, dans une position diamétralement opposée à la zone de charnière **60**, il est prévu des moyens de clipsage **58,59** permettant de maintenir les deux portions serrées l'une contre l'autre. Les moyens de clipsage **58,59** peuvent prendre la forme d'un crochet ou d'un clip déformable ou de toute autre coopération de forme destinée à obtenir une rétention mutuelle des pièces.

Une troisième cloison radiale **63**, opposée à la première cloison 61, permet d'obturer l'espace entre le tube et la zone couverte par le capot de protection, de manière à éviter que des objets ou des corps étrangers ne pénètrent dans l'espace intérieur du capot de protection.

Une fois que le capot de protection est installé autour du tube et du collier de serrage, tout endommagement par contact avec une partie agressive du collier 3 peut être avantageusement évité par la présence du capot de protection 5.
En particulier, si la connexion vient à contacter des éléments externes **90**, ceux-ci pourront venir en contact avec le capot de protection 5, mais ne pourront pas venir contacter directement le collier de serrage 3, comme ceci ressort de la figure 2.

Bien que généralement il n'y ait pas d'indexation du capot de protection 5 à une position angulaire particulière autour de l'axe A, il ne serait pas exclu de prévoir optionnellement une fonction anti-rotation pour le capot 5. A cet effet, on pourrait avoir un ergot radial saillant sur la première collerette **70** et plusieurs encoches correspondantes dans la troisième cloison radiale **63** (cf Fig 1). On ne pourrait correctement refermer la capot 5 sur le connecteur 1 que si l'encoche est en face d'un ergot, après quoi la rotation du capot autour de l'axe A est empêchée.

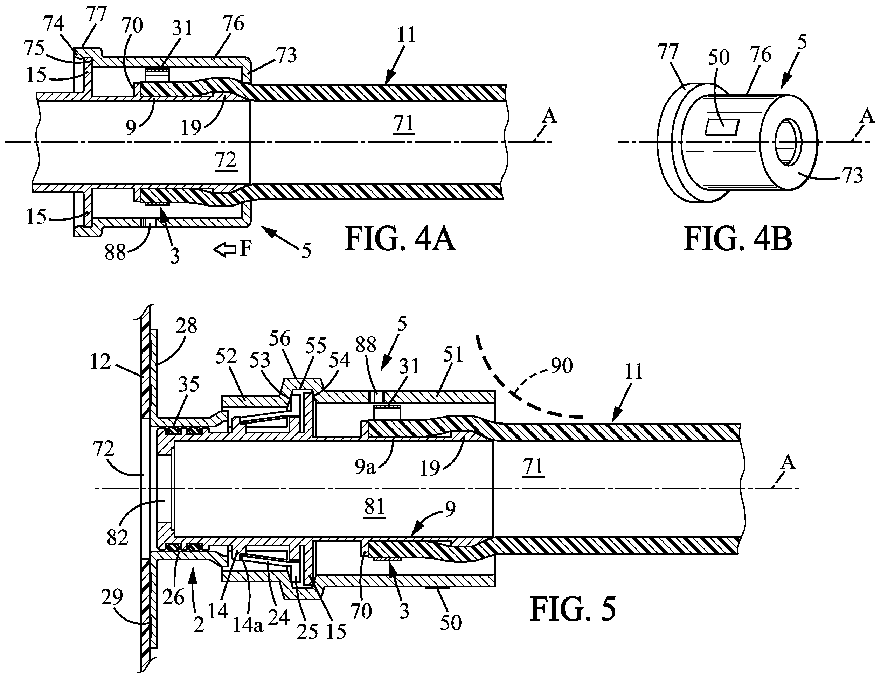

Dans un second mode de réalisation représenté aux figures 4A et 4B, le capot de protection **5** se présente comme une pièce monobloc de révolution autour de l'axe **A**, formée dans une matière plastique identique ou similaire à celles déjà évoquées pour le capot de protection du premier mode de réalisation.
Le capot de protection **5** comporte dans l'exemple illustré une portion principale cylindrique **76** destinée à s'interposer dans la position de protection, entre le collier de serrage et un élément externe 90. Le capot de protection **5** comporte en outre un rebord **77** frontal situé en avant de la portion principale dans le sens d'engagement du capot (flèche **F**) ; ce rebord forme un épaulement radial **75** et comprend au moins un clip flexible **74** sur son extrémité avant.
Le capot de protection **5** est placé sur le tube en attente avant l'assemblage du dispositif dans une zone arrière par rapport à l'extrémité avant du tube.
Le premier connecteur comprend outre la première collerette **70** une collerette principale **15** annulaire, de dimension plus importante que la première collerette, agencée dans une position plus éloignée de l'extrémité **1a** de l'embout 9.
Une fois que le tube souple a été inséré sur l'embout et que le collier de serrage a été posé et serré, on fait glisser le capot de protection d'arrière en avant en direction de la collerette principale **15**.
L'épaulement radial **75** susmentionné du capot vient buter contre une face de la collerette principale 15.
Les formes de clip **74** viennent s'accrocher sur l'autre face de la collerette principale 15, de manière à immobiliser axialement le capot de protection par rapport au premier connecteur 1.
En outre, de façon optionnelle, le capot de protection **5** peut comporter une cloison annulaire radiale arrière **73** destinée à isoler l'espace interne délimité par le capot et le tube souple. Ainsi, grâce à la collerette principale 15 et à cette cloison arrière 73 on évite que des corps étrangers puissent entrer dans la zone couverte par le capot. Dans ce deuxième mode de réalisation, il n'y a pas d'indexation du capot de protection à une position angulaire particulière autour de l'axe A ; toutefois on pourrait aussi prévoir une fonction anti-rotation similaire à ce qui a été décrit pour le premier mode de réalisation ci-dessus.
Dans un troisième mode de réalisation représentée à la figure 5, le premier connecteur est prévu pour s'interfacer avec le second connecteur **2** similaire à celui déjà décrit plus haut, le second connecteur comprend aussi une collerette principale **25** de diamètre identique ou voisin de la collerette principale **15** du premier connecteur **1**.

Selon un aspect optionnel représenté à la figure 5, lorsque le premier connecteur est prévu pour se raccorder sur le second connecteur il peut être prévu des languettes de verrouillage **24** dans le corps du second connecteur. Ces languettes de verrouillage 24, au nombre de deux dans l'exemple illustré sont flexibles, elles s'écartent vers l'extérieur au moment où la première collerette **14** avance au niveau de leur extrémité libre, puis reviennent vers l'intérieur pour se positionner en face d'une butée anti retrait **14a** sur l'arrière de la première collerette 14. Après quoi il n'est pas possible de faire marche arrière c'est-à-dire de retirer le premier connecteur du second connecteur sans au préalable effacer d'une façon ou d'une autre les languettes de verrouillage 24.

Dans ce troisième mode, le capot de protection 5 est de type à insertion radiale formé en deux demi-portions que l'on peut refermer l'une sur l'autre comme déjà indiqué concernant le premier mode de réalisation, sans indexation du capot 5 de protection à une quelconque position angulaire particulière autour de l'axe A .

Avantageusement, le capot comprend une projection radiale extérieure annulaire **56** avec en correspondance en vis-à-vis intérieurement une rainure interne **55** annulaire apte à former, en position de couplage, un verrouillage immobilisant les collerettes **15,25** adjacentes l'une contre l'autre comme ceci ressort de la figure 5. La bonne installation du capot de protection 5 fournit une indication de la bonne position de couplage des premiers et seconds connecteurs.
De plus, cette rainure interne 55 peut comprendre au moins une portion tronconique **53,54** (de section de forme trapézoïdale) de manière à exercer une pression axiale sur les collerettes, ce qui tend à rapprocher les deux connecteurs, ce qui peut représenter un sur-verrouillage par rapport à l'effet de clipsage de la languette flexible 24 susmentionnée.
En complément, pour faciliter le mouvement de fermeture des deux demi-portions du capot sur les collerettes, la partie postérieure radialement extérieure des collerettes pourrait comprendre un chanfrein qui faciliterait l'accostage des portions tronconiques 53,54.

En outre, le capot de protection **5** peut être tel qu'il forme des éléments de protection vis-à-vis du collier de serrage **3** sur le premier connecteur **1**.
Plus précisément, le capot de protection comprend une première extension axiale **51** du côté du premier connecteur et une seconde extension axiale **52** du côté du second connecteur.
La première extension axiale **51** forme une protection vis-à-vis du collier de serrage **3** : en effet celui-ci, en position de couplage, se retrouve dans la zone intérieure définie par cette extension axiale **51**. Moyennant quoi, si lors des manipulations ou déplacements du dispositif de connexion, ce dernier peut venir contacter des éléments externes **90**, alors ce n'est pas le collier de serrage qui viendra contacter lesdits éléments externes 90 mais au lieu de cela, ce sera le capot de protection 5 ici en l'occurrence l'extension axiale 51 qui viendra contacter le ou les éléments externes **90** (voir figure 5).
Ainsi, tout endommagement par contact avec une partie agressive du collier 3 peut être avantageusement évité.
Si le second connecteur est raccordé à un second tube souple, les mêmes dispositions et avantages sont obtenus, mutatis mutandis, du côté du second connecteur vis-à-vis du collier de serrage serrant le second tube sur l'embout du second connecteur, et dans ce cas le capot de protection peut être avantageusement symétrique par rapport au plan d'interface des collerettes **15,25**.
Il faut remarquer que le premier connecteur 1 délimite un premier passage creux 81 destiné à être mis en communication fluidique avec le premier espace 71. De même, le second connecteur délimite le second passage creux 82 déjà évoqué, destiné à être mis en communication fluidique avec le second espace 72 (l'intérieur de la poche plastique dans le cas particulier illustré).
Selon une caractéristique optionnelle, le dispositif de connexion 10 pourrait comprendre une fonction anti rotation pour empêcher le premier connecteur de tourner par rapport au second connecteur autour de l'axe A. Plus précisément, il pourrait s'agir par exemple un ergot radial (non représenté) agencé sur le premier connecteur par exemple au niveau de la collerette principale 15 ou bien encore au niveau de la première collerette 14. Cet ergot serait alors reçu dans une encoche ménagée dans la collerette 25 du second connecteur ou dans le corps 21 du second connecteur.

En outre, dans tous les modes de réalisation, il peut être prévu dans le corps du capot un orifice **88** traversant (autrement dit une lumière) qui permet une vérification visuelle de la présence du collier même lorsque le capot a été installé. Bien sûr la lumière peut être de taille différente, et il peut y avoir plusieurs lumières autour du corps du capot.

En outre, il est prévu une caractéristique optionnelle compatible avec toutes les variantes de réalisation précédemment évoquées : il s'agit de l'intégration d'un identifiant **50**, de type code barre ou étiquette électronique par exemple de type RFID, ou bien encore un code couleur. De façon préférée, on dispose cet identifiant sur le capot de protection qui est facile d'accès depuis l'extérieur. Dans la solution RFID, l'identifiant pourraient être dans n'importe quelle partie du premier connecteur.

## Revendications

1. Dispositif de connexion fluidique (10) apte et destiné à raccorder une première paroi délimitant un premier espace fluidique (71), en forme de tube souple (11), à une seconde paroi délimitant un second espace fluidique (72), en forme de tube ou d'enceinte souple ou rigide à usage unique, dans un ensemble biopharmaceutique, de sorte à être apte et destiné à assurer une communication fluidique entre le premier espace fluidique et le second espace fluidique, comprenant :
- un premier connecteur (1) délimitant un premier passage creux (81), apte et destiné à être raccordé à la première paroi et en communication fluidique avec le premier espace,
le premier connecteur comprenant un embout tubulaire (9) apte et destiné à recevoir le tube par insertion selon l'axe A,
- un collier de serrage (3) étant apte et destiné à être disposé autour de l'extrémité du tube (11) pour serrer ledit tube sur l'embout tubulaire du premier connecteur,
- un capot de protection (5) formant une pièce distincte, apte à être clipsé dans une position active où ledit capot se trouve au moins partiellement autour du premier connecteur et de son collier de serrage,
moyennant quoi le collier de serrage ne peut pas entrer en contact directement avec des éléments externes (90),
**caractérisé en ce que** le capot de protection est clipsé après serrage du collier, selon une orientation quelconque autour de l'axe A.

2. Dispositif selon la revendication 1, dans lequel le capot de protection est formé par deux demi-portions (5a,5b) configurées pour se refermer l'une sur l'autre dans la direction radiale autour du collier de serrage.

3. Dispositif selon la revendication 2, dans lequel le capot de protection comprend deux portions hémicylindriques complémentaires reliées par une portion souple de charnière (60), et les deux portions étant aptes à être clipsées l'une à l'autre dans une zone diamétralement opposée à la zone de charnière.

4. Dispositif selon l'une des revendications 2-3, dans lequel le capot de protection comprend des pattes radiales flexibles (65,64) destinées à venir s'appuyer sur le tube souple, dans une zone entre la position du collier et l'extrémité avant du tube (11 a).

5. Dispositif selon l'une des revendications 2-4, dans lequel le capot de protection comprend des cloisons radiales (61,62,63) pour encadrer le collier de serrage et ainsi immobiliser ou limiter le mouvement du capot le long de la direction axiale.

6. Dispositif selon la revendication 1, dans lequel le capot de protection est monobloc de révolution, le capot de protection étant disposé en attente en arrière sur le tube avant l'insertion du tube sur l'embout tubulaire (9), puis déplacé axialement vers l'avant pour venir dans une position active où il recouvre le collier de serrage.

7. Dispositif selon la revendication 6, dans lequel le capot de protection comporte une portion avant (77) apte à être clipsée sur une collerette (15) du premier connecteur dans la position active du capot de protection du collier.

8. Dispositif selon l'une des revendications 6-7, dans lequel le capot de protection comporte une portion arrière (73) apte à venir en appui sur le tube au voisinage de l'extrémité de l'embout du connecteur dans la position active du capot.

9. Dispositif selon l'une des revendications 1-8, comprenant en outre un second connecteur (2) délimitant un second passage creux (82), apte et destiné à être raccordé à la seconde paroi et en communication fluidique avec le second espace,
le premier connecteur et le second connecteur étant aptes et destinés à être couplés mutuellement dans une position de couplage relative selon un axe A, et dans lequel le capot de protection (5) est apte à être clipsé au moins partiellement autour du premier connecteur et autour du deuxième connecteur, pour former un témoin de bon couplage.

10. Dispositif selon la revendication 9 dans lequel le capot comprend une rainure intérieure (55) annulaire qui forme en position de couplage un moyen de verrouillage immobilisant des collerettes (15,25) adjacentes appartenant respectivement aux premier et deuxième connecteurs, formant témoin de bon montage.

11. Dispositif selon la revendication 10, dans lequel la rainure interne (55) forme au moins une portion tronconique de manière à exercer une pression axiale sur les collerettes, ce qui tend à rapprocher les deux connecteurs.

12. Dispositif selon l'une des revendications 9-11, dans lequel, le second connecteur comprend au moins une languette flexible (24) adaptée pour venir se clipser sur une seconde collerette (14) du premier connecteur de manière à former ainsi un premier verrouillage de la position de couplage préalable à l'installation du capot de protection (5).

13. Dispositif selon l'une des revendications 1-12, dans lequel le deuxième connecteur comprend en outre un identifiant (50) de type code barre ou étiquette RFID ou code couleur disposé sur le capot de protection.

14. Dispositif selon l'une des revendications 1-13, dans lequel le capot comprend une lumière permettant de vérifier la présence du collier une fois le capot installé.

15. Ensemble biopharmaceutique comprenant un dispositif de connexion fluidique selon l'une des revendications 1 à 14.

## Patentansprüche

1. Fluidverbindungsvorrichtung (10), die geeignet und dazu bestimmt ist, eine erste Wandung, die einen ersten Fluidraum (71), in Form einer flexiblen Röhre (11) definiert, mit einer zweiten Wandung zu verbinden, die einen zweiten Fluidraum (72), in Form eines Rohres oder eines flexiblen oder starren Behälters für den einmaligen Gebrauch definiert, in einer biopharmazeutischen Anordnung, die geeignet und dazu bestimmt ist, die Fluidverbindung zwischen dem ersten Fluidraum und dem zweiten Fluidraum sicherzustellen, umfassend:
- einen ersten Anschluss (1), der eine erste hohle Durchführung (81) definiert, die geeignet und dazu bestimmt ist, mit der ersten Wandung verbunden zu werden und in Fluidverbindung mit dem ersten Raum zu stehen, wobei der erste Anschluss ein rohrförmiges Endstück (9) umfasst, das geeignet und dazu bestimmt ist, das Rohr durch Einführen entlang der Achse A aufzunehmen,
- eine Schlauchklemme (3), die geeignet und dazu bestimmt ist, um das Rohrende (11) herum angeordnet zu werden, um das Rohr an dem rohrförmigen Endstück des ersten Anschlusses festzuklemmen,
- eine Schutzabdeckung (5), die ein separates Teil bildet und geeignet ist, in einer aktiven Position angeklipst zu werden, in der die Abdeckung zumindest teilweise um den ersten Anschluss und seine Schlauchklemme angeordnet ist, wobei die Schlauchklemme nicht in direkten Kontakt mit externen Elementen (90) kommen kann, **dadurch gekennzeichnet, dass** die Schutzabdeckung nach dem Festklemmen der Schlauchklemme in beliebiger Orientierung um die Achse A angeklipst wird.

2. Vorrichtung nach Anspruch 1, wobei die Schutzabdeckung aus zwei Hälften gebildet ist (5a, 5b), die konfiguriert sind, um sich aufeinander in radialer Richtung um die Schlauchklemme zu schließen.

3. Vorrichtung nach Anspruch 2, wobei die Schutzabdeckung zwei komplementäre halbzylindrische Teile aufweist, die durch einen flexiblen Gelenkteil (60) verbunden sind, und die beiden Teile geeignet sind, in einem dem Scharnierbereich genau entgegengesetzten Bereich zusammengeklipst zu werden.

4. Vorrichtung nach einem der Ansprüche 2-3, wobei die Schutzabdeckung flexible radiale Laschen (65, 64) umfasst, die dazu bestimmt sind, , in einem Bereich zwischen der Position der Klemme und dem vorderen Ende der Röhre (11a) auf den flexiblen Schlauch zu drücken.

5. Vorrichtung nach einem der Ansprüche 2-4, wobei die Schutzabdeckung radiale Trennwände (61, 62, 63) umfasst, um die Schlauchklemme zu umschließen und dadurch die Bewegung der Abdeckung entlang der axialen Richtung zu blockieren oder zu begrenzen.

6. Vorrichtung nach Anspruch 1, wobei die Schutzabdeckung aus einem Rotationsstück besteht, wobei die Schutzabdeckung vor dem Einführen des Rohres in das rohrförmige Endstück (9) in Warteposition hinter dem Rohr angeordnet ist und dann axial nach vorne bewegt wird, um in eine aktive Position zu kommen, wo sie die Schlauchklemme abdeckt.

7. Vorrichtung nach Anspruch 6, wobei die Schutzabdeckung einen vorderen Teil (77) umfasst, der geeignet ist, in der aktiven Position der Schutzabdeckung der Klemme auf einen Flansch (15) des ersten Anschlusses geklipst zu werden.

8. Vorrichtung nach einem der Ansprüche 6-7, wobei die Schutzabdeckung einen hinteren Teil (73) umfasst, der geeignet ist, in der aktiven Position der Abdeckung zum Aufliegen auf das Rohr angrenzende Ende des Endstücks des Anschlusses zu kommen.

9. Vorrichtung nach einem der Ansprüche 1-8, ferner umfassend einen zweiten Anschluss (2), der eine zweite hohle Durchführung (82) definiert, die geeignet und dazu bestimmt ist, mit der ersten Wandung verbunden zu werden und in Fluidverbindung mit dem zweiten Raum zu stehen, wobei der erste Anschluss und der zweite Anschluss geeignet und dazu bestimmt sind, miteinander in einer relativen Kopplungsposition entlang einer Achse A gekoppelt zu sein, und wobei die Schutzabdeckung (5) geeignet und dazu bestimmt ist, zumindest teilweise um den ersten Anschluss und um den zweiten Anschluss geklipst zu werden, um einen Indikator für eine gute Kopplung zu bilden.

10. Vorrichtung nach Anspruch 9, wobei die Abdeckung eine ringförmige innere Nut (55) umfasst, die in Kopplungsposition ein Verriegelungsmittel bildet, das die jeweils zu den ersten und zweiten Anschlüssen gehörenden angrenzenden Flansche (15, 25) blockiert und einen Indikator für eine gute Kopplung zu bildet.

11. Vorrichtung nach Anspruch 10, wobei die innere Nut (55) mindestens einen kegelstumpfartigen Teil bildet, um einen axialen Druck auf die Flansche auszuüben, was die beiden Anschlüsse letztlich einander näher bringt.

12. Vorrichtung nach einem der Ansprüche 9-11, wobei der zweite Anschluss mindestens eine flexible Feder (24) umfasst, die geeignet ist, sich auf einen zweiten Flansch (14) des ersten Anschlusses anklipsen zu lassen, um dadurch eine erste Verriegelung der Kopplungsposition vor der Installation die Abdeckung (5) zu bilden.

13. Vorrichtung nach einem der Ansprüche 1-12, wobei der zweite Anschluss ferner eine Kennung (50) vom Typ eines Strichcodes oder RFID-Etiketts oder Farbcodes beinhaltet, die auf der Schutzabdeckung angeordnet ist.

14. Vorrichtung nach einem der Ansprüche 1-13, wobei die Abdeckung eine Öffnung beinhaltet, der die Überprüfung des Vorhandenseins der Klemme erlaubt, sobald die Abdeckung installiert ist.

15. Biopharmazeutische Anordnung, umfassend eine Fluidverbindungsvorrichtung nach einem der Ansprüche 1 bis 14.

## Claims

1. Fluid-connection device adapted and intended for connecting a first wall defining a first fluid space (71), in the form of a flexible pipe (11), to a second wall defining a second fluid space (72), in the form of a pipe or enclosure that is flexible or rigid and disposable, in a biopharmaceutical assembly, for ensuring fluid communication between the first fluid space and the second fluid space, comprising:
- a first connector (1) defining a first hollow passage (81), adapted and intended for connection to the first wall and in fluid communication with the first space,
the first connector comprising a tubular nozzle (9) adapted and intended for receiving the pipe by insertion along the axis A,
- a pipe clamp (3) adapted and intended for placement around the end of the pipe (11) in order to clamp said pipe onto the tubular nozzle of the first connector,
- a protective cover (5) forming a separate part, suitable for being snap-fitted into an operative position in which said cover is at least partially surrounding the first connector and its pipe clamp,
by means of which the pipe clamp cannot come into direct contact with external elements (90),
**characterized in that** the protective cover being snap-fitted into place in any orientation about the axis A after the clamp is tightened.

2. Device according to claim 1, wherein the protective cover is formed by two half-portions (5a, 5b) configured to come together around the clamp in the radial direction.

3. Device according to claim 2, wherein the protective cover comprises two complementary semi-cylindrical portions connected by a flexible hinge portion (60), and the two portions are adapted to snap-fit together in an area diametrically opposite the hinge area.

4. Device according to one of claims 2-3, wherein the protective cover comprises flexible radial tabs (65,64) intended to bear against the flexible pipe in an area between the position of the clamp and the front end of the pipe (11 a).

5. Device according to one of claims 2-4, wherein the protective cover comprises radial walls (61,62,63) to enclose the pipe clamp and thereby immobilize or limit movement of the cover in the axial direction.

6. Device according to claim 1, wherein the protective cover is a single part that is rotationally symmetrical, the protective cover being placed in an inoperative rearward position on the pipe prior to insertion of the pipe onto the tubular nozzle (9), then moved axially forward into an operative position where it covers the pipe clamp.

7. Device according to claim 6, wherein the protective cover comprises a front portion (77) adapted to be snap-fitted onto a collar (15) of the first connector when the protective cover is in the operative position on the clamp.

8. Device according to one of claims 6-7, wherein the protective cover comprises a rear portion (73) adapted to bear against the pipe adjacent to the end of the nozzle of the connector when the cover is in the operative position.

9. Device according to one of claims 1-8, further comprising a second connector (2) defining a second hollow passage (82), adapted and intended for connection to the second wall and in fluid communication with the second space,
the first connector and the second connector being adapted and intended to be coupled together in a relative coupling position along an axis A, and the protective cover (5) being suitable for snap-fitting into place so as to at least partially surround the first connector and second connector, forming an indicator of proper coupling.

10. Device according to claim 9, wherein the cover comprises an annular inner groove (55) which, in the coupling position, forms a locking means immobilizing the adjacent collars (15,25) that are respectively part of the first and second connectors, forming an indicator of proper assembly.

11. Device according to claim 10, wherein the inner groove (55) forms at least one tapered portion so as to exert an axial pressure on the collars, which tends to bring the two connectors together.

12. Device according to one of claims 9-11, wherein the second connector comprises at least one flexible tab (24) suitable for snap-fitting onto a second collar (14) of the first connector, thereby establishing a first locking in the coupling position prior to installation of the protective cover (5).

13. Device according to one of claims 1-12, wherein the second connector further comprises an identifier (50) such as a barcode or RFID tag or color code provided on the protective cover.

14. Device according to one of claims 1-13, wherein the cover comprises an opening enabling verification of the presence of the clamp once the cover is installed.

15. Biopharmaceutical assembly comprising a fluid-connection device according to one of claims 1 to 14.
